# DEMANDE DE BREVET EUROPEEN

(11) **EP 1 477 143 A1**
(43) Date de publication de la demande: **17.11.2004**
(21) Numéro de dépôt: 04356069.7
(22) Date de dépôt: 12.05.2004
(51) Int. Cl.: A61F 2/38

(54) **Jeu d'éléments prothétiques pour un ensemble prothétique tibial**

(30) Priorité: 12.05.2003 FR 0305683
(71) Demandeur: Tornier, 38330 Saint Ismier (FR)
(72) Inventeur: Tornier, Alain, 38330 Saint Ismier (FR); Dejour, David, 69006 Lyon (FR); Bonnin, Michel, 69340 Francheville (FR)
(74) Mandataire: Myon, Gérard

(57) **Abrégé**

Un ensemble prothétique tibial comporte généralement une embase (6) destinée à être solidarisée à un tibia et un patin adapté pour être monté de manière fixe sur l'embase. Ce patin délimite une surface articulaire prothétique destinée à coopérer avec un élément fémoral (2) et présentant une épine antérieure (56) de part et d'autre de laquelle sont situées deux cavités glénoïdiennes (54, 55) de réception de condyles fémoraux (14, 15).

Pour former un tel ensemble, le chirurgien dispose d'un jeu comportant l'embase (6) et plusieurs patins qui sont montables selon respectivement des positions angulaires propres dans chacune desquelles l'axe longitudinal de l'épine antérieure (56) de chaque patin forme un angle prédéterminé avec l'axe médian antéro-postérieur de l'embase (6).

Application au domaine des prothèses de l'articulation du genou, notamment humain.

## Description

La présente invention concerne un jeu d'éléments prothétiques pour un ensemble prothétique tibial, lequel ensemble tibial comporte une embase destinée à être solidarisée à un tibia, ainsi qu'un patin à même d'être monté sur l'embase et délimitant une surface articulaire prothétique destinée à coopérer avec un élément fémoral prothétique.

Elle s'applique au domaine des prothèses partielles ou totales de l'articulation du genou, notamment humain.

Il existe deux grandes familles d'ensembles prothétiques tibiaux selon que le patin, monté sur l'embase, est ou non mobile, notamment en rotation, par rapport à l'embase. Si le patin est fixe par rapport à l'embase, on parle d'ensembles tibiaux à simple articulation, c'est-à-dire d'ensembles permettant l'articulation entre uniquement le patin et l'élément fémoral, tandis que, si le patin est mobile par rapport à l'embase, on parle d'ensembles tibiaux à double articulation ou d'ensembles rotatoires.

L'invention concerne des éléments prothétiques pour former un ensemble tibial à simple articulation.

Pour poser un tel ensemble tibial, le chirurgien doit dans un premier temps, de façon connue en soi, solidariser fermement l'embase à l'extrémité supérieure du tibia. Ensuite, le patin est fixé à l'embase, par exemple par clipsage, vissage et/ou collage, dans une position prédéterminée par rapport à l'embase.

Il ressort cependant de la pratique chirurgicale que, selon l'anatomie particulière de chaque patient et/ou selon quel genou, droit ou gauche, est opéré, la position du patin imposée par l'embase et le composant fémoral est rarement la mieux adaptée, c'est-à-dire celle permettant au patient de recouvrer un comportement articulaire proche du comportement anatomique. C'est d'ailleurs une des raisons pour laquelle se sont développés des ensembles prothétiques à double articulation évoqués plus haut, mais qui restent plus coûteux et sont mécaniquement moins stables.

Pour autoriser une certaine liberté de positionnement du patin en cours de pose par rapport à l'embase, on a proposé par le passé d'interposer entre l'embase et le patin des cales à géométrie particulière. Cette solution est cependant complexe à mettre en oeuvre pour le chirurgien en raison du nombre important à la fois de constituants de l'ensemble prothétique et de gestes opératoires nécessaires pour, en fin d'intervention, fixer les positions relatives de ces constituants.

Le but de l'invention est de proposer un jeu d'éléments prothétiques pour former un ensemble prothétique tibial à simple articulation, qui permette d'obtenir un ensemble tibial plus compact et plus conforme à l'anatomie du patient, sans pour autant compliquer le travail de pose du chirurgien, ni augmenter significativement le coût de cet ensemble.

A cet effet, l'invention a pour objet un jeu d'éléments prothétiques pour un ensemble prothétique tibial, comportant une embase destinée à être solidarisée à l'extrémité supérieure d'un tibia et définissant un axe médian antéro-postérieur, ledit ensemble tibial comprenant ladite embase et un patin adapté pour être monté de manière fixe sur l'embase et délimitant une face articulaire prothétique tibiale destinée à coopérer avec un élément fémoral prothétique. Cette surface articulaire présente à la fois deux cavités glénoïdiennes de réception de condyles interne et externe de l'élément fémoral et une épine antérieure délimitant longitudinalement un axe de part et d'autre duquel sont situées ces cavités glénoïdiennes. Ce jeu comporte plusieurs patins adaptés pour être montés de manière fixe sur l'embase selon des positions angulaires respectives dans chacune desquelles l'axe de l'épine antérieure de chaque patin forme, avec l'axe médian de l'embase, un angle prédéterminé, au moins l'un de ces angles prédéterminés étant non nul et un autre étant éventuellement nul.

Le chirurgien, en disposant de l'embase d'un tel jeu et en choisissant un patin dans ce jeu, peut ainsi poser un ensemble prothétique mieux approprié à l'anatomie du patient, tout en corrigeant éventuellement un mauvais positionnement angulaire de l'embase par rapport au tibia.

Suivant d'autres caractéristiques de ce jeu, prises isolément ou selon toutes les combinaisons techniquement possibles :
- les patins du jeu sont choisis parmi des patins dont les axes des épines forment avec l'axe médian de l'embase des angles dont les valeurs respectives sont comprises entre 0 et 15° environ ;
- les angles formés entre les axes des épines des patins du jeu et l'axe médian de l'embase valent respectivement 0, 2, 4, 6 et 8° environ ;
- chaque patin du jeu est pourvu d'une face de montage opposée à la surface articulaire, ladite face portant des moyens de fixation sur l'embase tibiale adaptés pour coopérer avec cette embase par complémentarité de formes ;
- les faces de montage des patins du jeu sont sensiblement identiques les unes aux autres ;
- les moyens de fixation de chaque patin du jeu comporte une lèvre de clipsage dans une rainure sensiblement complémentaire portée par l'embase ;
- l'embase comporte un rebord périphérique et le contour périphérique de la face de montage de chaque patin du jeu est adapté pour s'inscrire à l'intérieur du rebord de l'embase ;
- au moins des parties correspondantes du contour intérieur du rebord et du contour périphérique de la face de montage de chaque patins du jeu sont adaptées pour coopérer par complémentarité de formes en tant que moyens de fixation ; et
- la surface articulaire de chaque patin du jeu présente une troisième cavité glénoïdienne s'étendant suivant l'axe de l'épine antérieure et destinée à recevoir un troisième condyle de l'élément fémoral.

L'invention vise également un procédé de pose d'un ensemble prothétique tibial pour une articulation du genou, dans lequel :
- on dispose d'un jeu d'éléments prothétiques comprenant, d'une part, une embase destinée à être solidarisée à l'extrémité supérieure d'un tibia et définissant un axe médian antéro-postérieur et, d'autre part, plusieurs patins adaptés chacun pour être fixés sur l'embase et délimitant chacun une surface articulaire prothétique tibiale destinée à coopérer avec un élément fémoral prothétique, laquelle surface articulaire de chaque patin présente à la fois deux cavités glénoïdiennes de réception de condyles interne et externe de l'élément fémoral et une épine antérieure délimitant longitudinalement un axe de part et d'autre duquel sont situées ces cavités glénoïdiennes, les différents patins du jeu étant adaptés pour être montés sur l'embase selon des positions angulaires respectives dans chacune desquelles l'axe de l'épine antérieure de chaque patin forme, avec l'axe médian de l'embase, un angle prédéterminé éventuellement nul,
- on résèque la partie supérieure d'un tibia et on solidarise à cette extrémité réséquée l'embase ou une embase fantôme de même géométrie que l'embase à implanter,
- on choisit un patin parmi les patins de ce jeu, et
- on monte de manière fixe le patin choisi sur l'embase, après avoir si nécessaire remplacer l'embase fantôme par l'embase à implanter.

Suivant une caractéristique avantageuse de ce procédé, pour choisir le patin à monter parmi les patins du jeu, et après avoir solidarisé l'embase à implanter ou l'embase fantôme sur le tibia :
- on monte sur cette embase un patin fantôme adapté pour être retenu de manière mobile sur l'embase et délimitant une surface articulaire prothétique tibiale destinée à coopérer avec l'élément fémoral anatomique ou prothétique, laquelle surface articulaire du patin fantôme présente une épine antérieure définissant un axe longitudinal,
- on entraîne l'articulation du genou suivant au moins un mouvement de flexion-extension,
- on détermine le décalage angulaire résiduel entre l'axe médian de l'embase et l'axe longitudinal de l'épine antérieure du patin fantôme, et
- on choisit, parmi les patins du jeu, le patin dont l'angle prédéterminé est sensiblement égal ou le plus proche de la valeur du décalage angulaire déterminé à l'aide du patin fantôme.

L'invention sera mieux comprise à la lecture de la description qui va suivre, donnée uniquement à titre d'exemple et faite en référence aux dessins sur lesquels :
- la figure 1 est une vue en perspective d'une prothèse totale de genou comportant un ensemble prothétique tibial formé à partir d'un jeu d'éléments prothétiques selon l'invention ;
- la figure 2 est une vue en perspective de l'embase de l'ensemble prothétique tibial représenté sur la figure 1 ;
- la figure 3 est une vue en perspective, sous un angle différent de la figure 2, du patin de l'ensemble prothétique tibial représenté sur la figure 1 ;
- la figure 4 est une vue en élévation du patin de la figure 3, prise selon la flèche IV indiquée sur la figure 3 ;
- la figure 5 est une vue en élévation, prise selon la flèche V à la figure 4, du patin et, une coupe médiane de l'élément fémoral de la figure 1 ; et
- les figure 6A, 6B et 6C sont des vues analogues à la figure 4 et illustrent respectivement d'autres patins du jeu d'éléments prothétiques selon l'invention.

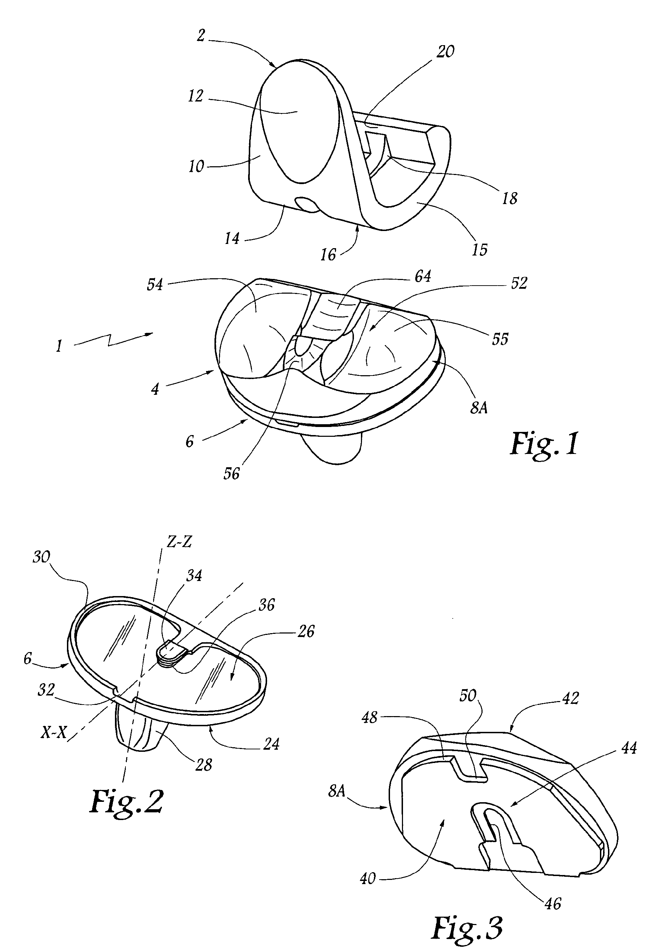

Sur la figure 1 est représentée une prothèse totale 1 d'une articulation de genou humain. Cette prothèse comprend d'une part un élément fémoral prothétique 2 destiné à être solidarisé à l'extrémité inférieure du fémur d'un patient, et d'autre part un ensemble prothétique tibial 4 comportant une embase 6 destinée à être solidarisée à l'extrémité supérieure du tibia du patient et un patin 8A adapté pour être monté de manière fixe sur l'embase. Comme il sera expliqué en détail plus loin, le patin 8A est issu d'une série de plusieurs patins.

Dans toute la suite, les termes « antérieur », « postérieur », « interne » et « externe » s'entendent dans leur sens anatomique, c'est-à-dire par rapport au fémur ou au tibia du squelette humain lorsque ces os sont sensiblement alignés, c'est-à-dire dans une position d'extension de l'articulation du genou.

Plus précisément, l'élément fémoral 2 comporte une platine antérieure 10 délimitant une face avant 12 destinée à coopérer avec la rotule du patient. La platine 10 est prolongée vers l'arrière par deux condyles interne 14 et externe 15 formant une surface articulaire fémorale prothétique 16. Les deux condyles sont séparés l'un de l'autre par une rainure traversante 18 s'étendant selon une direction antéro-postérieure. Les parties d'extrémités postérieures des deux condyles sont raccordées par un pontet 20 qui s'étend transversalement à la direction longitudinale de la rainure, en en formant la paroi de fond arrière.

L'embase 6 de l'ensemble tibial 4, représentée plus en détail sur la figure 2, comporte une platine 22 essentiellement plane et dont les faces inférieure et supérieure sont respectivement référencées 24 et 26. Cette platine définit un axe médian antéro-postérieur X-X, le plan antéro-postérieur comportant cet axe formant un plan de symétrie pour l'embase.

Sur sa face inférieure 24, l'embase 6 est pourvue d'un tenon 28 s'étendant suivant un axe longitudinal Z-Z sensiblement perpendiculaire au plan de la platine 22. Ce tenon est destiné à être introduit dans le canal médullaire tibial et y être retenu, par exemple par du ciment.

Sur sa face supérieure 26, l'embase 6 comporte un rebord périphérique saillant 30 interrompu dans sa partie avant par une échancrure 32. Dans sa partie postérieure, la face supérieure 26 délimite un doigt saillant 34, s'étendant longitudinalement suivant l'axe X-X. Le chant latéral du doigt est de contour arrondi et délimite une rainure périphérique 36.

Le patin 8A, représenté plus en détail sur les figures 3 à 5, est une pièce monobloc, réalisée en matière plastique, par exemple en polyéthylène. Il présente une face inférieure 40 et une face supérieure 42.

La face inférieure 40 est pourvue de moyens 44 de fixation du patin sur l'embase 6, adaptés pour permettre la mise en place et la retenue de manière fixe du patin par rapport à l'embase. Comme représenté sur la figure 3, ces moyens 44 comporte une lèvre 46 de profil arrondi complémentaire du profil du doigt 34, à même d'être clipsée dans la rainure 36 de l'embase 6. En outre, le contour extérieur 48 de la face inférieure 40 est inscrit à l'intérieur du contour formé par le rebord 30 de l'embase 6, la partie avant du contour 48 étant sensiblement complémentaire de la partie correspondante du contour du rebord 30. De la sorte, une fois le patin mis en place sur l'embase, la coopération de formes à la fois entre le doigt 34 et la lèvre 46 et entre les parties avant du contour extérieur 48 et du contour intérieur du rebord 30 assure l'immobilité du patin 8A par rapport à l'embase 6.

La face inférieure 40 du patin 8A comporte dans sa partie avant une encoche 50 adaptée pour être disposée sensiblement dans le prolongement de l'échancrure 32 du rebord 30 lorsque le patin est mis en place sur l'embase. Par introduction de la lame ou de la pointe d'un ancillaire approprié à l'intérieur de l'échancrure 32 et de l'encoche 50, il est possible d'appliquer sur la face inférieure du patin un effort de levier dirigé vers le haut, afin de désolidariser en force le patin 8A de l'embase 6. Une telle opération intervient notamment lors de la dépose de l'ensemble tibial 4, par exemple en fin de vie de cet ensemble.

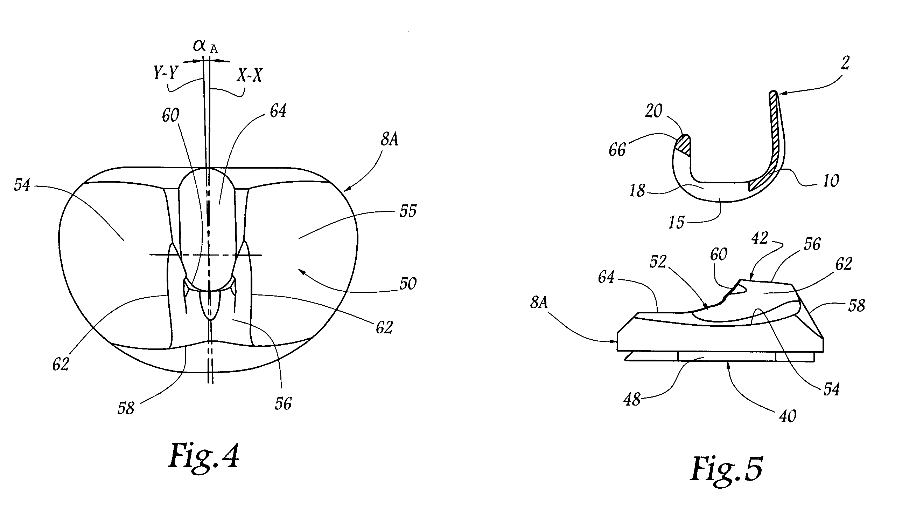

La face supérieure 42 du patin 8A forme une surface articulaire tibiale prothétique 52, représentée en détail sur les figures 4 et 5, destinée à coopérer avec la face articulaire fémorale 16. A cet effet, cette surface 52 délimite deux cavités glénoïdiennes principales 54 et 55, destinées chacune à recevoir un des condyles interne 14 et externe 15 de l'élément fémoral 2.

Une épine antérieure saillante 56 est formée entre ces deux cavités. L'épine 56 est destinée à être logée dans la rainure 18 de l'élément fémoral 2 de façon à participer au guidage des faces articulaires 16 et 52 l'une par rapport à l'autre.

Cette épine présente, dans la direction antéro-postérieure, un axe longitudinal Y-Y formant un axe de symétrie pour les cavités glénoïdiennes 54 et 55. Comme indiqué sur la figure 4, lorsque le patin 8A est monté sur l'embase 6, l'axe Y-Y forme avec l'axe médian antéro-postérieur X-X de l'embase 6 un angle noté α_{A}, valant environ 2°.

La face avant 58 de l'épine forme une butée pour la paroi de fond avant de la rainure 18 séparant les deux condyles. En d'autres termes, cette surface avant 58 est destinée à retenir l'élément fémoral 2 par rapport à l'ensemble tibial 4 lorsque l'articulation du genou est sollicitée en hyper-extension.

La face arrière 60 de l'épine 56 forme quant à elle une surface de butée pour le pontet 20 reliant les deux condyles 14 et 15. En d'autres termes, cette surface arrière bloque l'élément fémoral 2 par rapport à l'ensemble tibial 4 lorsque l'articulation du genou est sollicitée en hyper-flexion.

Les faces latérales 62 de l'épine 56 limitent quant à elles le degré de liberté rotatoire entre l'élément fémoral 2 et l'ensemble tibial 4, la géométrie de ces surfaces 62 étant étudiée pour permettre la reproduction aussi fidèle que possible des mouvements anatomiques correspondants.

En arrière et dans le prolongement de l'épine antérieure 56, la surface articulaire tibiale 52 du patin 8A délimite une troisième cavité glénoïdienne 64 destinée à recevoir la surface postérieure 66 du pontet 20 qui forme de la sorte un troisième condyle fémoral. Selon le profil concave de cette cavité 64, le comportement en flexion de l'articulation est rendu plus fidèle au comportement anatomique. Pour le patin 8A représenté, l'axe Y-Y de l'épine 56 forme un axe de symétrie pour la cavité glénoïdienne 64.

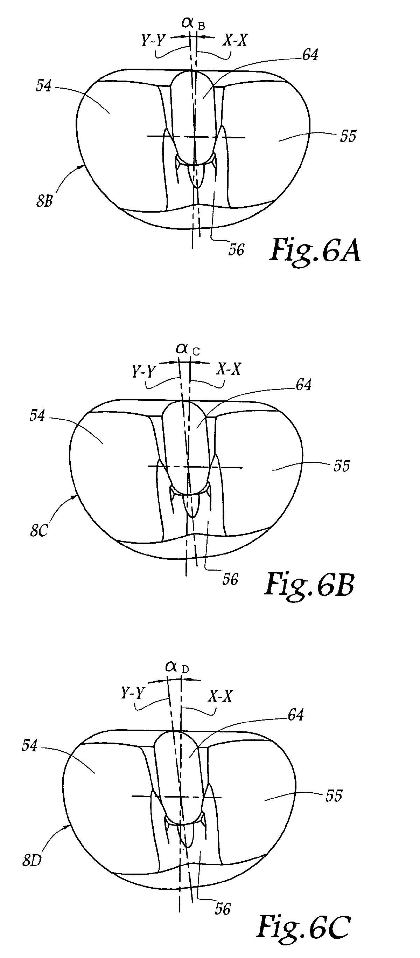

Comme évoqué précédemment, le patin 8A des figures 1 et 3 à 5 appartient à une série de plusieurs patins tibiaux se distinguant essentiellement les uns des autres par la valeur de l'angle formé entre l'axe médian X-X de l'embase 6 et l'axe Y-Y de chaque patin lorsque ces patins sont montés sur l'embase par l'intermédiaire des moyens de fixation 44. Un jeu d'éléments prothétiques est formé par l'embase 6 et la série de plusieurs patins du type du patin 8A. Plus précisément, le jeu considéré à titre d'exemple comporte à la fois le patin 8A et des patins 8B, 8C et 8D représentés sur les figures 6A à 6C, ainsi qu'éventuellement d'autres patins non représentés.

Les faces inférieures de chacun des patins du jeu précité sont sensiblement identiques. Elles sont toutes adaptées pour être individuellement montées de manière fixe sur la seule embase 6 et comportent toutes à cet effet une lèvre de clipsage de forme et d'orientation identiques à la lèvre 46 détaillée pour le patin 8A. Seul le contour extérieur de ces faces inférieures varie légèrement d'un patin à l'autre, pour ne pas gêner la mise en place de ces faces à l'intérieur du contour délimité par le rebord 30 de l'embase 6.

Les faces supérieures de chacun des patins du jeu comportent les mêmes éléments que ceux décrits pour le patin 8A, à savoir deux cavités glénoïdiennes principales 54 et 55, une épine antérieure 56 et une cavité glénoïdienne secondaire 64. Cependant, la disposition d'ensemble de ces éléments varie d'un patin à l'autre : l'axe Y-Y de chaque patin 8A à 8D forme avec l'axe X-X de l'embase 6 un angle noté α_{A}, α_{B}, α_{C}, α_{D}, les valeurs respectives de ces angles étant d'environ 2, 4, 6 et 8°.

La pose à rotule luxée d'un ensemble tibial prothétique à partir de l'embase 6 et de la série de patins 8A à 8D est la suivante.

Après avoir réséqué la partie d'extrémité supérieure du tibia, et éventuellement creusé les parois internes du canal médullaire tibial, le tenon 28 de l'embase 6, ou en variante un tenon analogue d'une embase fantôme de même géométrie que l'embase 6 ultérieurement implantée, est logé et immobilisé rigidement dans ce canal médullaire, en étant éventuellement cimenté.

Le chirurgien place ensuite un patin fantôme à même d'être retenu sur l'embase tout en conservant par rapport à cette dernière au moins un degré de liberté en rotation suivant un axe sensiblement parallèle à l'axe Z-Z. Ce patin fantôme délimite une surface articulaire tibiale analogue à la surface articulaire 52 détaillée précédemment pour le patin 8A et présentant notamment une épine antérieure analogue à l'épine 56 pour le patin 8A. Par entraînement de l'articulation du genou suivant un ou plusieurs mouvements d'extention-flexion-extension, le chirurgien provoque le déplacement, notamment en rotation, du patin fantôme par rapport à l'embase. Une fois que le genou est repositionné en position d'extension, le chirurgien mesure le décalage angulaire résiduel entre l'axe X-X de l'embase 6 et l'axe longitudinal de l'épine antérieure du patin fantôme, par exemple au moyen de graduations portées par le chant avant du patin par rapport à un repère porté par le chant avant de l'embase. Selon la morphologie du patient, la pathologie nécessitant la mise en place d'un ensemble prothétique tibial et/ou le positionnement de l'embase par rapport au tibia réalisé par le chirurgien, cet angle résiduel peut prendre toute valeur comprise entre zéro et environ une quinzaine de degrés.

Le chirurgien choisit alors parmi les patins 8A à 8D du jeu précité dont il dispose le patin dont l'angle de décalage α_{A}, α_{B}, α_{C} ou α_{D} est le plus proche de l'angle mesuré, puis, après avoir retiré le patin fantôme, met en place le patin choisi sur l'embase 6 par l'intermédiaire des moyens de fixation 44.

Grâce aux patins du jeu selon l'invention, le chirurgien est ainsi à même de sélectionner rapidement le patin fixe le plus approprié pour le patient en cours d'opération. Le coût de l'ensemble prothétique tibial finalement posé est sensiblement identique à celui des ensembles traditionnels employés jusqu'alors, les patins non utilisés du jeu pouvant être stockés et utilisés pour une intervention ultérieure sur un autre patient.

Bien que le jeu décrit précédemment comportent quatre patins dont les décalages respectifs valent 2, 4, 6 et 8°, on comprendra que l'invention vise des jeux comprenant au moins deux patins qui possèdent des décalages angulaires respectifs différents l'un de l'autre, l'un de ses patins pouvant présenté un axe Y-Y sensiblement aligné avec l'axe X-X de l'embase 6, c'est-à-dire présenté un angle de décalage sensiblement nul.

Divers aménagements et variantes au jeu décrit ci-dessus sont en outre envisageables :
- pour renforcer la solidarisation de l'embase 6 au tibia, des trous traversants peuvent être prévus dans la platine 22 afin de recevoir des vis d'ancrage dans l'os, ces vis étant mises en place depuis la face supérieure 26 de la platine avant que le patin ne soit monté ;
- la surface inférieure 24 de la platine 22 peut présenter un profil en creux et en bosses, destiné à favoriser l'immobilisation de l'embase par rapport au tibia ; et/ou
- le tenon 28 de l'embase 6 peut être pourvu d'un orifice taraudé d'axe Z-Z afin de permettre le vissage d'une tige d'ancrage rapportée à introduire plus profondément dans le canal médullaire tibial.

## Revendications

1. Jeu d'éléments prothétiques pour un ensemble prothétique tibial (4), comportant une embase (6) destinée à être solidarisée à l'extrémité supérieure d'un tibia et définissant un axe médian antéro-postérieur (X-X), ledit ensemble tibial comprenant ladite embase (6) et un patin adapté pour être monté de manière fixe sur l'embase (6) et délimitant une face articulaire prothétique tibiale (52) destinée à coopérer avec un élément fémoral prothétique (2), laquelle surface articulaire présente à la fois deux cavités glénoïdiennes (54, 55) de réception de condyles interne (14) et externe (15) de l'élément fémoral (2) et une épine antérieure (56) délimitant longitudinalement un axe (Y-Y) de part et d'autre duquel sont situées ces cavités glénoïdiennes, **caractérisé en ce que** ledit jeu comporte plusieurs patins (8A, 8B, 8C, 8D) adaptés pour être montés de manière fixe sur l'embase (6) selon des positions angulaires respectives dans chacune desquelles l'axe (Y-Y) de l'épine antérieure (56) de chaque patin forme, avec l'axe médian (X-X) de l'embase, un angle prédéterminé (α_{A}, α_{B}, α_{C}, α_{D}), au moins l'un de ces angles prédéterminés étant non nul et un autre étant éventuellement nul.

2. Jeu suivant la revendication 1, **caractérisé en ce que** les patins (8A, 8B, 8C, 8D) du jeu sont choisis parmi des patins dont les axes (Y-Y) des épines (56) forment avec l'axe médian (X-X) de l'embase (6) des angles (α_{A}, α_{B}, α_{C}, α_{D}) dont les valeurs respectives sont comprises entre 0 et 15° environ.

3. Jeu suivant la revendication 2, **caractérisé en ce que** lesdits angles (α_{A}, α_{B}, α_{C}, α_{D}) formés entre les axes (Y-Y) des épines (56) des patins (8A, 8B, 8C, 8D) du jeu et l'axe médian (X-X) de l'embase (6) valent respectivement 0, 2, 4, 6 et 8° environ.

4. Jeu suivant l'une quelconque des revendications précédentes, **caractérisé en ce que** chaque patin (8A, 8B, 8C, 8D) du jeu est pourvu d'une face de montage (40) opposée à la surface articulaire (52), ladite face portant des moyens (44) de fixation sur l'embase tibiale (6) adaptés pour coopérer avec cette embase par complémentarité de formes.

5. Jeu suivant la revendication 4, **caractérisé en ce que** les faces de montage (40) des patins (8A, 8B, 8C, 8D) du jeu sont sensiblement identiques les unes aux autres.

6. Jeu suivant la revendication 4 ou 5, **caractérisé en ce que** les moyens de fixation (44) de chaque patin (8A, 8B, 8C, 8D) du jeu comporte une lèvre (46) de clipsage dans une rainure (36) sensiblement complémentaire portée par l'embase (6).

7. Jeu suivant l'une quelconque des revendications 4 à 6, **caractérisé en ce que** l'embase (6) comporte un rebord périphérique (30) et **en ce que** le contour périphérique (48) de la face de montage (40) de chaque patin (8A, 8B, 8C, 8D) du jeu est adapté pour s'inscrire à l'intérieur du rebord (30) de l'embase (6).

8. Jeu suivant la revendication 7, **caractérisé en ce qu'**au moins des parties correspondantes du contour intérieur du rebord (30) et du contour périphérique (48) de la face de montage (40) de chaque patins (8A, 8B, 8C, 8D) du jeu sont adaptées pour coopérer par complémentarité de formes en tant que moyens de fixation (44).

9. Jeu suivant l'une quelconque des revendications précédentes, **caractérisé en ce que** la surface articulaire (52) de chaque patin (8A, 8B, 8C, 8D) du jeu présente une troisième cavité glénoïdienne (64) s'étendant suivant l'axe (Y-Y) de l'épine antérieure (56) et destinée à recevoir un troisième condyle de l'élément fémoral (2).
